# EUROPÄISCHE PATENTSCHRIFT

(11) **EP 2 508 126 B1**
(45) Veröffentlichungstag und Bekanntmachung des Hinweises auf die Patenterteilung: **05.03.2014**
(21) Anmeldenummer: 11002926.1
(22) Anmeldetag: 07.04.2011
(51) Int. Cl.: A61B 5/103, A61B 5/107

(54) **Verwendung eines Druckmesssystems zur Ermittlung des Sitzknochenabstands eines menschlichen Beckens**
Use of a pressure measuring system for determining the distance between the sitting bones of a human pelvis
Usage d'un système de mesure de pression pour l'établissement de la distance de l'os iliaque d'un bassin humain

(43) Veröffentlichungstag der Anmeldung: 10.10.2012
(73) Patentinhaber: SQlab GmbH, 82024 Taufkirchen (DE)
(72) Erfinder: Hild, Tobias, 82024 Taufkirchen (DE)
(74) Vertreter: Koelle, Alexander

(56) Entgegenhaltungen:
- WO-A1-2007/029358
- DE-U1-202006 008 296
- US-A1- 2006 225 297
- US-A1- 2009 124 935

## Beschreibung

### Technisches Gebiet

Die Erfindung betrifft die Verwendung eines Druckmesssystems zur Visualisierung und Ermittlung des individuellen Sitzknochenabstands eines menschlichen Beckens, in dem der zu messende Körper oder Gegenstand auf ein Blatt Papier oder ähnlichem, das sich auf einer Grundplatte mit Noppen (z.B. in Zuckerhut-, Kreiszylinder-, Kreiskegel-, Pyramiden-, Tetraederform) die vorzugsweise in regelmäßigen und symmetrischen Abständen auf der Grundplatte angeordnet sind, befindet, aufgebracht wird.

Mit Hilfe des visuell dargestellten Drucks und Druckverlaufs lässt sich der individuelle Sitzknochenabstand einer Person ermitteln und dadurch ist es möglich, einen für die Person bzw. für diesen Sitzknochenabstand geeigneten Sattel auszuwählen. Insofern kann diese Erfindung auch als Verkaufshilfe für den Fachhandel dienen. Unter anderem ist es dadurch möglich - nämlich mittels des ermittelten Sitzknochenabstands - die Sitzauflagen (Pads) des ergonomischen Sattels nach DE 20 2004 014 467 U1 entsprechend anzuordnen bzw. auf dem Sattel anzubringen oder einen entsprechenden ergonomischen Sattel nach DE 20 2005 013749.9 U1, DE 20 2007 008 321.1 U1 oder EP 2 003 046 B1 in der jeweiligen Sattelbreite auszuwählen.

### Stand der Technik

Bekannt ist die Ermittlung des Sitzknochenabstandes eines menschlichen Beckens mittels Druckmessfolien, Messpappen oder Messschablonen. Mit Hilfe dieser war der Fachverkäufer bislang in der Lage, die maximalen Druckpunkte des menschlichen Beckens zu definieren und den Abstand der Sitzknochen zu erkennen und zu vermessen sowie im Anschluss daran den für den Kunden optimalen Sattel auszuwählen und/oder zu empfehlen.

Bei den Messpappen handelt es sich um handelsübliche Wellpappen. Auf eine derartige Wellpappe setzt sich der Kunde und die Beckenknochen-Eindrücke auf der Wellpappe geben das Breitenmaß des Sitzknochenabstands wieder.

Nachteil dieser Messpappen ist, dass sie sich nicht in ausreichendem Maße zur Visualisierung eignen, da sie sich unter Druck nur zusammendrücken und einer gesonderten Anschaffung bedürfen, was einen nicht unerheblichen Kostenfaktor für den Fahrradfachhandel darstellt.

Weiterer Nachteil dieser Druckmesssysteme ist, dass sich die erzielten Messergebnisse nicht oder nur unzureichend digitalisieren und speichern lassen.

Bekannt sind ferner elektronische Druckmesssysteme und Druckmessfolien, die zwar ebenfalls in der Lage sind mittels Druckmessbildern und einer 3-D und 2-D Animation die Druckpunkte und die Druckverteilung visuell darzustellen, jedoch sind derartige Druckmessfolien in der Anschaffung verhältnismäßig teuer und ferner auch kompliziert zu bedienen.

Bekannt ist aus DE 20 2006 008 296.4 U1 ein Druckmesssystem zur Visualisierung des von einem menschlichen Körper oder einem Gegenstand ausgehenden Drucks, insbesondere zur visuellen Darstellung und Ermittlung des individuellen Sitzknochenabstands eines menschlichen Beckens, bestehend aus einem (Sitz-)Kissen, das mit einer farbigen Flüssigkeit oder einem farbigen Gel gefüllt ist. Wird von oben Druck durch den zu messenden Körper oder Gegenstand auf die übereinander liegenden Schichten aus (Sitz-)Kissen ausgeübt, so wird an den Stellen hohen Drucks die Flüssigkeit oder das Gel zur Seite gedrückt, wobei sich die Farbe einerseits aufhellt. Andererseits entstehen auch Stellen, an denen das (Sitz-)Kissen vollständig transparent wird. Es ergeben sich Farbverläufe durch unterschiedliche Farbintensitäten. Die hellste Stelle ist die Stelle mit dem größten Druck. Dadurch kann der Sitzknochenabstand ermittelt werden, sobald der Kunde sich auf die Vorrichtung gesetzt hat. Nachteil dieses Druckmesssystem ist, dass es zu aufwendig und zu teuer in der Herstellung und Anschaffung ist.

Sinn und Zweck der Ermittlung des Sitzknochenabstandes ist eine Druckentlastung in der Weise, dass sich die Körperlast hauptsächlich über die Sitzbeinhöcker des menschlichen Beckens auf die Satteloberfläche ablastet. Bei herkömmlichen Sattelformen und ohne Ermittlung des Sitzknochenabstands lastet bei Männern auf den Dammbereich und bei Frauen im Bereich des Schambeins ein zu hoher Druck. Es besteht die Gefahr, dass beim Mann der hohe Druck im Dammbereich negative Auswirkungen auf die sexuelle Leistungsfähigkeit des Mannes haben kann. Bei Frauen liegt das Schambein etwas tiefer als beim Mann, so dass dieses meistens schmerzhaft auf die Sattelnase drückt. In der Vergangenheit gab es verschiedene Lösungsansätze verschiedener Sattelhersteller, den Druck im Dammbereich oder im Bereich des Schambeins zu senken, z.B. durch die Verwendung einer weichen Sattelnase, mittels Verwendung von Gels oder von Aussparungen und Löchern im Sattel. Sättel mit derartigen Merkmalen sind bereits aus EP 1 394 025 und aus US 5 356 205 bekannt geworden. Mit diesen Sätteln kann jedoch eine noch nicht ausreichende Druckentlastung im Dammbereich bzw. Schambeinbereich erreicht werden. Insbesondere mit Sätteln nach DE 20 2005 013 749 U1 und DE 20 2004 014 467 U1 lässt sich bereits eine erhebliche Druckentlastung im Dammbereich bzw. Schambeinbereich erreichen.

Aus WO 2007/029358 A1 ist eine Vorrichtung zur Ermittlung eines Fußabdrucks bekannt. Nachteil ist jedoch, dass hier die Folie oder das Blatt Papier nicht direkt den Fußabdruck auweist und festhält, so dass es nicht für weitere Auswertungen verwendet werden kann. Der Abdruck ist allenfalls auf der Grundplatte abzulesen oder erkennbar. Dies würde jedoch bei der Messung des Sitzknochenabstandes nicht nur die Auswertung erschweren, sondern auch die Lagerung und Archivierung des Messergebnisses. Ferner ist das Messverfahren dadurch zu kostenintensiv. Das Messergebnis ist nicht direkt auf der Folie oder dem Blatt Papier vorhanden. Darüber hinaus sind bei dem Messverfahren nach WO 2007/029358 A1 noch weitere Farbpartikel o.ä. zur Markierung und Visualisierung des Fußabdrucks auf der Bodenplatte erforderlich. Dies ist zu aufwendig und kompliziert sowohl in der Vorbereitung der Messung als auch beim Messvorgang. Diese Vorrichtung eignet sich nicht für die Verwendung zur Messung des Sitzknochenabstandes eines menschlichen Beckens. Mit dieser Vorrichtung können nur Konturen eines Körpers oder eines Körpterteils abgebildet werden.

Ferner ist aus US 2006/0225297 A1 eine Vorrichtung zur Ermittlung eines Fußabdrucks bekannt. Nachteil ist, dass dabei eine Vielzahl von beweglichen Messstiften sowie ein Luftkissen unterhalb der Messstifte erforderlich sind. Dadurch ist die Messvorrichtung sehr komplex und teuer in der Herstellung. Auch lässt sich das Messergebnis nicht auf einfache Weise auf einem Blatt Papier ablesen. Des Weiteren erfolgt auch keine dauerhafte Ermittlung des Fußabdrucks auf der Messfolie. Diese Vorrichtung eignet sich daher auch nicht für die Verwendung zur Messung des Sitzknochenabstandes eines menschlichen Beckens. Mit dieser Vorrichtung können ebenfalls nur Konturen eines Körpers oder eines Körpterteils abgebildet werden.

### Offenlegung der Erfindung

Aufgabe der vorliegenden Erfindung ist es, den Druck und die Druckverteilung eines menschlichen Beckens oder aber auch eines anderen Gegenstands auf einfachste und kostengünstige Art und Weise dauerhaft visuell und leicht archivierbar darzustellen, so dass diese Druckmessvorrichtung insbesondere im Sportfachhandel, vorzugsweise im Radsportfachhandel, zur Sattelberatung eingesetzt werden kann. Mit Hilfe dieser visuellen Darstellung lässt sich problemlos und einfach der Sitzknochenabstand des menschlichen Beckens ermitteln und ein optimaler Sattel für den Kunden auswählen und/oder empfehlen, der den Druck bei Männern im Dammbereich bzw. bei Frauen im Bereich des Schambeins senkt.

Die Aufgabe der vorliegenden Verwendung wird erfindungsgemäß durch die kennzeichnenden Merkmale des ersten Patentanspruchs gelöst. Durch die Merkmale der Unteransprüche wird die Erfindung vorteilhaft ausgestaltet bzw. modifiziert.

Die Erfindung besteht aus der Verwendung eines Blatt Papiers oder ähnlichem und einer Grundplatte mit Noppen. Diese Noppen können beispielsweise in Kreiskegel-, sog. Zuckerhut-, Pyramiden-, Tetraeder- oder Kreiszylinderform ausgestaltet sein. Bei der sog. Zuckerhutform handelt es sich um einen Kreiskegelstumpf mit einem Kugelabschnitts- oder Kalottenkopf.

Wird von oben Druck durch den zu messenden Körper oder Gegenstand auf die übereinander liegenden Schichten aus dem Blatt Papier und der Grundplatte ausgeübt, so drücken die Noppen an Stellen hohen Drucks stark auf das Papier, verformen es und erzeugten Vertiefungen, wobei teilweise die Noppen auch durch das Blatt Papier treten können, aber nicht müssen. Wenn sich ein Mensch auf das Blatt Papier und die Grundplatte setzt, so zeigen die erzeugten Vertiefungen und/oder Löcher in dem Blatt Papier dauerhaft den Sitzknockenabstand der betreffenden Person an. Diese Vertiefungen und/oder Löcher in dem Blatt Papier stellen das Messergebnis dar. Dieses Blatt Papier lässt sich dauerhaft lagern und archivieren. Aufgrund der Löcher in dem Blatt Papier ist es auch nach längerer Zeit noch einem Beweis bzw. Nachweis zugänglich.

Bei den vorteilhaften Verwendungsvarianten sind die Noppen in sog. Zuckerhutform oder in anderen Formgestaltungen ausgestaltet, z.B. Kreiskegel-, Pyramiden-, Tetraeder- oder Kreiszylinderform.

Mit Hilfe einer speziell für diesen Zweck entwickelten Sattelberatungssoftware kann der Fachhändler aufgrund des somit ermittelten Sitzknochenabstands des Beckens des Kunden diesem Kunden den für ihn optimal passenden Sattel empfehlen. Insbesondere mit ergonomischen Sätteln, z.B. nach DE 20 2004 014 467 U1, DE 20 2005 013749.9 U1, DE 20 2007 008 321.1 U1 oder EP 2 003 046 B1, lassen sich zusammen mit dem Gegenstand der Erfindung maximale Druckentlastungen beim Mann im Dammbereich bzw. bei der Frau im Schambeinbereich erreichen, da der für den jeweiligen Sitzknochenabstand passende Sattel mit entsprechender Sattelbreite ausgewählt werden kann oder bei einem Sattel nach DE 20 2004 014 647 U1 die Sitzauflagen (Pads) entsprechend auf dem Sattel angeordnet und positioniert werden können.

In den Zeichnungen zeigen:

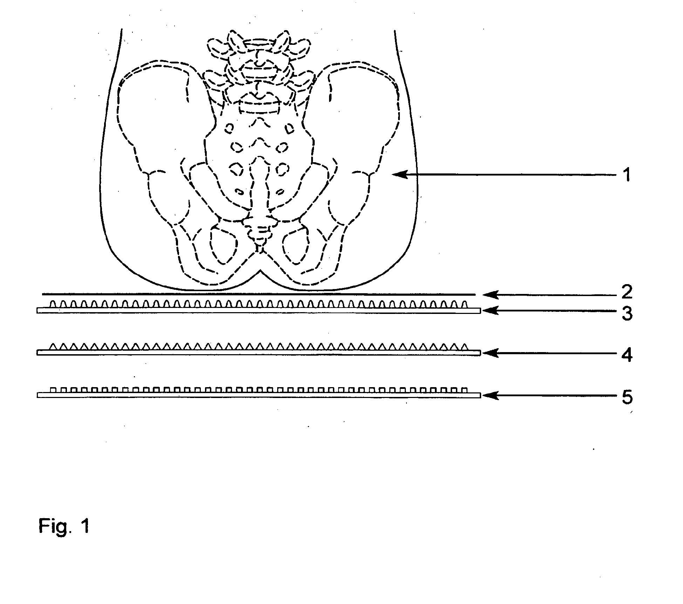

Fig. 1 eine schematische Seitenansicht der Druckmessvorrichtung vor der Aufbringung der Körperlast 1 oder dem Gewicht eines Gegenstands auf das Blatt Papier 2 und die Grundplatte mit Noppen in sog. Zuckerhutform 3, in Pyramidenform 4 und in Kreiszylinderform 5

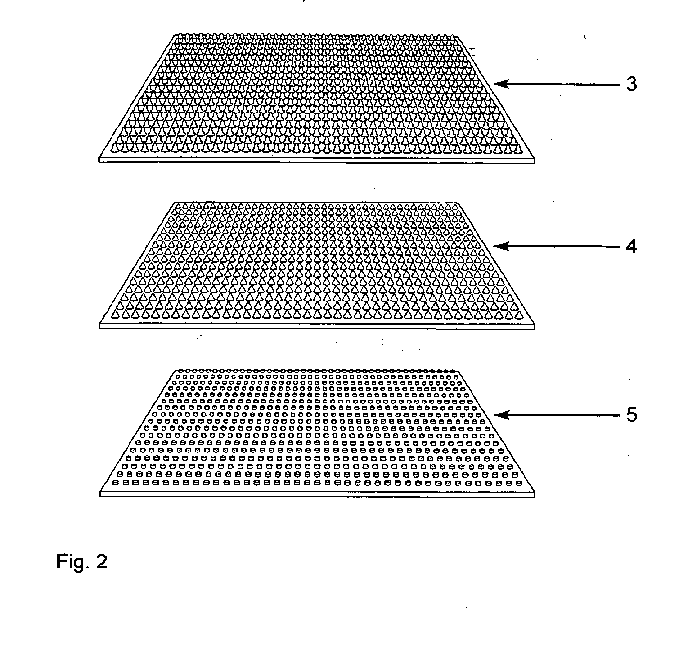

Fig. 2 eine perspektivische Draufsicht der verschiedenen Grundplatten mit Noppen in Zuckerhutform 3, in Pyramidenform 4 und in Kreiszylinderform 5

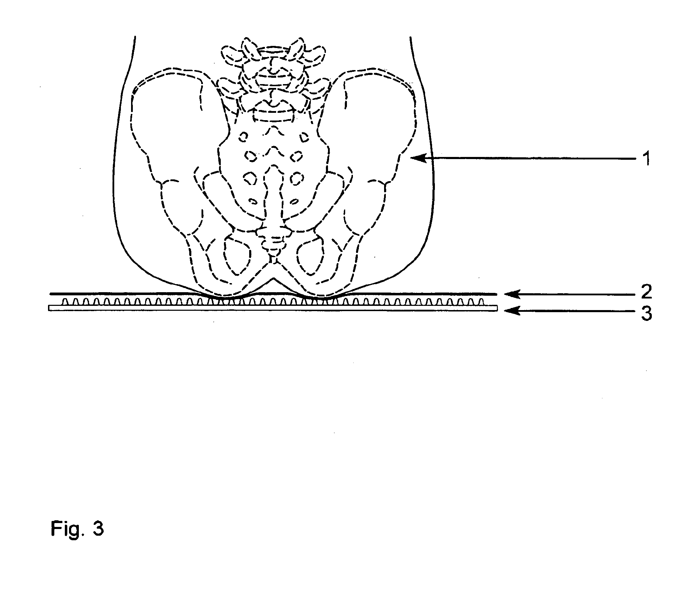

Fig. 3 eine schematische Seitenansicht auf die Druckmessvorrichtung nach der Aufbringung der Körperlast 1 oder dem Gewicht eines Gegenstands wobei gezeigt wird, dass sich an der Stelle der Sitzknochen die Noppen durch das Papier 2 drücken

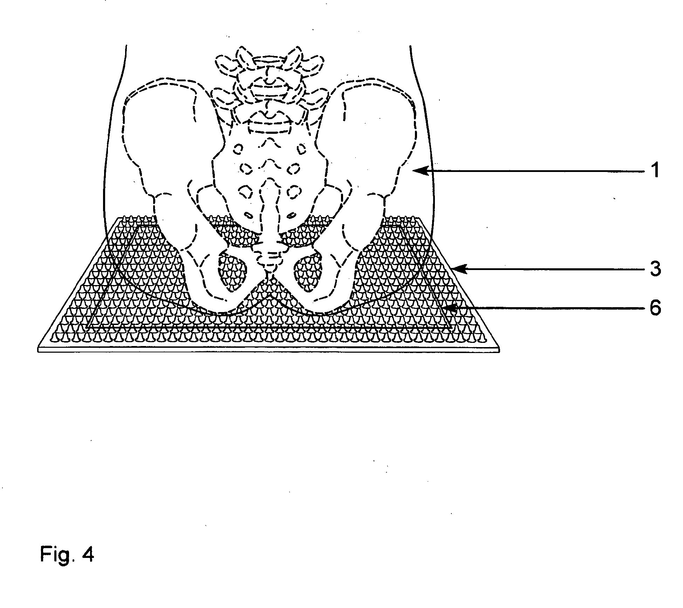

Fig. 4 eine persektivische Draufsicht der Druckmessvorrichtung vor der Aufbringung der Körperlast 1 oder dem Gewicht eines Gegenstands auf ein transparentes Blatt Papier 6 und die Grundplatte mit Noppen in sog. Zuckhutform 3

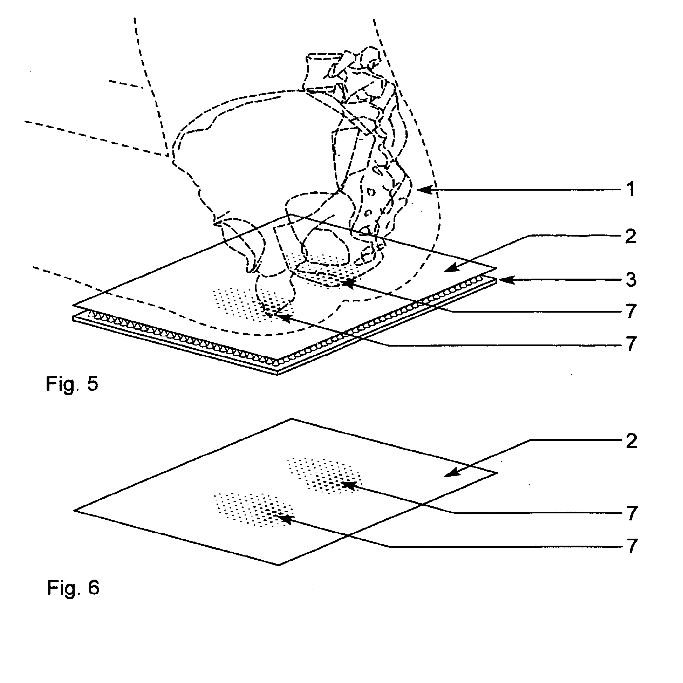

Fig. 5 eine perspektivische Draufsicht der Druckmessvorrichtung nach der Aufbringung der Körperlast 1 oder dem Gewicht eines Gegenstands wobei gezeigt wird, dass sich an der Stelle der Sitzknochen die Noppen durch das Papier 2 drücken und mit den Eindrucksstellen 7 das Messergebnis erzeugen
Fig. 6 eine perspektivische Draufsicht auf das Blatt Papier 2 mit dem Messergebnis 7, den Eindrucksstellen

### Bezugszeichenliste

- 1: Zu messender Körper oder Gegenstand
- 2: Blatt Papier oder ähnliches
- 3: Grundplatte mit Noppen in Form eines Kegelstumpfs mit Kugelabschnitts- oder Kalottenkopf (sog. Zuckerhutform)
- 4: Grundplatte mit Noppen in Kreiskegelform
- 5: Grundplatte mit Noppen in Kreiszylinderform
- 6: Transparentes Papier
- 7: Eindrücke in das Papier (Messergebnis)

## Patentansprüche

1. Verwendung einer Grundplatte (3), zur Ermittlung des Sitzknochenabstands eines menschlichen Beckens,
**dadurch gekennzeichnet, dass**
diese Grundplatte (3) Noppen enthält und ein Blatt Papier (2) verwendet wird, welches auf der Grundplatte mit Noppen angeordnet ist.

2. Verwendung nach Anspruch 1, **dadurch gekennzeichnet, dass** die Noppen der Grundplatte (3) in Form von Kreiszylindern ausgestaltet sind.

3. Verwendung nach Anspruch 1, **dadurch gekennzeichnet, dass** die Noppen der Grundplatte (3) in Form von Pyramiden ausgestaltet sind.

4. Verwendung nach Anspruch 1, **dadurch gekennzeichnet, dass** die Noppen der Grundplatte (3) in Form von Kreiskegeln ausgestaltet sind.

5. Verwendung nach Anspruch 1, **dadurch gekennzeichnet, dass** die Noppen der Grundplatte (3) in der Form von Kreiskegelstümpfen mit Kugelabschnitts- oder Kalottenköpfen ausgestaltet sind.

6. Verwendung nach Anspruch 1, **dadurch gekennzeichnet, dass** die Noppen der Grundplatte (3) in der Form von Tetraedern ausgestaltet sind.

7. Verwendung nach Anspruch 1, **dadurch gekennzeichnet, dass** die Noppen der Grundplatte (3) in der Form von Kreiszylindern mit Kreiskegelköpfen, z.B. Nägeln, ausgestaltet sind.

## Claims

1. The use of a base plate (3) for determining the sit bone width of a human pelvis, **characterised in that** this base plate (3) comprises nubs, and a sheet of paper (2) is used that is arranged on the base plate comprising nubs (3).

2. The use according to claim 1, **characterised in that** the nubs of the base plate (3) are designed in the shape of circular cylinders.

3. The use according to claim 1, **characterised in that** the nubs of the base plate (3) are designed in the shape of pyramids.

4. The use according to claim 1, **characterised in that** the nubs of the base plate (3) are designed in the shape of circular cones.

5. The use according to claim 1, **characterised in that** the nubs of the base plate (3) are designed in the shape of truncated circular cones with heads of spherical segments or heads of spherical caps.

6. The use according to claim 1, **characterised in that** the nubs of the base plate (3) are designed in the shape of tetrahedrons.

7. The use according to claim 1, **characterised in that** the nubs of the base plate (3) are designed in the shape of circular cylinders with circular-cone-shaped heads, e.g. nails.

## Revendications

1. Utilisation d'une plaque de base (3) pour calculer la distance des ischions d'un bassin humain, **caractérisée en ce que** cette plaque de base (3) comporte des picots et qu'une feuille de papier (2) est employée, laquelle est disposée sur la plaque de base (3) avec picots.

2. Utilisation selon la revendication 1, **caractérisée en ce que** les picots de la plaque de base (3) ont la forme de cylindres circulaires.

3. Utilisation selon la revendication 1, **caractérisée en ce que** les picots de la plaque de base (3) ont la forme de pyramides.

4. Utilisation selon la revendication 1, **caractérisée en ce que** les picots de la plaque de base (3) ont la forme de cônes de révolution.

5. Utilisation selon la revendication 1, **caractérisée en ce que** les picots de la plaque de base (3) ont la forme de cônes de révolution émoussés avec des têtes à segment sphérique ou calotte.

6. Utilisation selon la revendication 1, **caractérisée en ce que** les picots de la plaque de base (3) ont la forme de tétraèdres.

7. Utilisation selon la revendication 1, **caractérisée en ce que** les picots de la plaque de base (3) ont la forme de cylindres circulaires avec des têtes à cônes de révolution, par exemple des clous.
